(19) **Europäisches Patentamt**
**European Patent Office**
**Office européen des brevets**

(11) **EP 4 375 927 A1**

(12) **EUROPEAN PATENT APPLICATION**

(43) Date of publication:
**29.05.2024 Bulletin 2024/22**

(21) Application number: **22209876.6**

(22) Date of filing: **28.11.2022**

(51) International Patent Classification (IPC):
**G06T 7/33** *(2017.01)*  **G06T 7/35** *(2017.01)*
**A61B 6/03** *(2006.01)*

(52) Cooperative Patent Classification (CPC):
**G06T 7/33; A61B 6/5211; G06T 7/35;** A61B 6/032;
G06T 2207/10076; G06T 2207/10081;
G06T 2207/10088; G06T 2207/10132;
G06T 2207/20016; G06T 2207/20041;
G06T 2207/20081; G06T 2207/20084;
G06T 2207/30004; G06T 2207/30096

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
• **GHESU, Florin-Cristian**
**Skillman, NJ, 08558 (US)**

• **VIZITIU, Anamaria**
**520038 Sfantu Gheorghe, Covasna (RO)**

(74) Representative: **Horn Kleimann Waitzhofer**
**Schmid-Dreyer**
**Patent- und Rechtsanwälte PartG mbB**
**Theresienhöhe 12**
**80339 München (DE)**

Remarks:
Amended claims in accordance with Rule 137(2)
EPC.

(54) **LESION TRACKING IN 4D LONGITUDINAL IMAGING STUDIES**

(57)    Systems and methods for tracking an anatomical object in medical images are provided. A first input medical image and a second input medical image each depicting an anatomical object of a patient are received. The first input medical image comprises a point of interest corresponding to a location of the anatomical object. A first set of embeddings associated with a plurality of scales is extracted from the first input medical image using a machine learning based extraction network. The plurality of scales comprises a coarse scale, one or more intermediate scales, and a fine scale. A second set of embeddings associated with the plurality of scales is extracted from the second input medical image using the machine learning based extraction network. A location of the anatomical object in the second input medical image is determined by comparing embeddings of the first set of embeddings corresponding to the point of interest with embeddings of the second set of embeddings. The location of the anatomical object in the second input medical image is output.

FIG 2

200

Receive a first input medical image and a second input medical image each depicting an anatomical object of a patient, the first input medical image comprising a point of interest corresponding to a location of the anatomical object — 202

Extract, from the first input medical image, a first set of embeddings associated with a plurality of scales using a machine learning based extraction network — 202

Extract, from the second medical image, a second set of embeddings associated with the plurality of scales using the machine learning based extraction network — 202

Determine a location of the anatomical object in the second input medical image by comparing embeddings of the first set of embeddings corresponding to the point of interest with embeddings of the second set of embeddings — 202

Output the location of the anatomical object in the second input medical image — 202

**Description**

TECHNICAL FIELD

The present invention relates generally to tracking of anatomical objects in medical images, and in particular to lesion tracking in 4D (four dimensional) longitudinal imaging studies.

BACKGROUND

Longitudinal imaging studies are often used for monitoring anatomical changes of a patient over time. In one exemplary application, longitudinal imaging studies may be used for temporally monitoring lesions as such longitudinal imaging studies provide temporal information that comprehensively captures the dynamic changes in the lesions and provide valuable insight into the response of the lesions to treatment. Temporal monitoring of lesions typically involves detecting and tracking the lesions across the images of the longitudinal imaging studies. However, the development of reliable automatic lesion tracking is hindered by the complexity of the data, the absence of large, annotated datasets, and the difficulties associated with lesion identification due to, for example, varying sizes, poses, shapes, and sparsely distributed locations.

BRIEF SUMMARY OF THE INVENTION

In accordance with one or more embodiments, systems and methods for tracking an anatomical object in medical images are provided. A first input medical image and a second input medical image each depicting an anatomical object of a patient are received. The first input medical image comprises a point of interest corresponding to a location of the anatomical object. A first set of embeddings associated with a plurality of scales is extracted from the first input medical image using a machine learning based extraction network. The plurality of scales comprises a coarse scale, one or more intermediate scales, and a fine scale. A second set of embeddings associated with the plurality of scales is extracted from the second input medical image using the machine learning based extraction network. A location of the anatomical object in the second input medical image is determined by comparing embeddings of the first set of embeddings corresponding to the point of interest with embeddings of the second set of embeddings. The location of the anatomical object in the second input medical image is output.

In one embodiment, the machine learning based extraction network is trained based on anatomical landmarks identified in a pair of training images. In one embodiment, the machine learning based extraction network is trained with multi-task learning to perform one or more auxiliary tasks. The one or more auxiliary tasks may comprise at least one of landmark detection, segmentation, pixel-wise matching, or image reconstruction. In one embodiment, the machine learning based extraction network is trained with unlabeled and unpaired training images.

In one embodiment, matching embeddings of the second set of embeddings that are most similar to embeddings of the first set of embeddings corresponding to the point of interest are identified. The location of the anatomical object in the second input medical image is determined as a pixel or voxel that corresponds to the matching embeddings. In one embodiment, the location of the anatomical object in the second input medical image is determined by comparing embeddings of the first set of embeddings and the second set of embeddings with corresponding scales of the plurality of scales.

In one embodiment, the receiving, the extracting the first set of embeddings, the extracting the second set of embeddings, the determining, and the outputting are repeated using an additional input medical image as the second input medical image.

In one embodiment, the first input medical image and the second input medical image respectively comprise a baseline medical image and a follow-up medical image of a longitudinal imaging study of the patient.

It is understood that the method steps as disclosed above, below with respect to embodiments of this disclosure and/or as identified in the claims can be implemented in terms of dedicated processing means of an apparatus or by a processor suitable for carrying out the steps in a computer implemented fashion.

These and other advantages of the invention will be apparent to those of ordinary skill in the art by reference to the following detailed description and the accompanying drawings.

BRIEF DESCRIPTION OF THE DRAWINGS

Figure 1 shows a framework for training and applying a machine learning based extraction network for lesion tracking, in accordance with one or more embodiments;

Figure 2 shows a method for tracking an anatomical object in medical images using a machine learning based extraction network, in accordance with one or more embodiments;

Figure 3 shows a workflow for tracking an anatomical object in medical images using a machine learning based extraction network, in accordance with one or more embodiments;

Figure 4 shows a workflow for training a machine learning based extraction network, in accordance with one or more embodiments;

Figure 5 shows a workflow for training a machine learning based extraction network with an auxiliary task, in accordance with one or more embodiments;

Figure 6 shows an exemplary artificial neural network that may be used to implement one or more embodiments;

Figure 7 shows a convolutional neural network that may be used to implement one or more embodiments; and

Figure 8 shows a high-level block diagram of a computer that may be used to implement one or more embodiments.

DETAILED DESCRIPTION

The present invention generally relates to methods and systems for lesion tracking in 4D (four dimensional) longitudinal imaging studies. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa. Embodiments of the present invention are described herein with reference to the figures, where like reference numerals represent the same or similar elements.

Embodiments described herein provide for a lesion tracking framework for tracking lesions (or any other anatomical object) identified in a baseline medical image to a follow-up medical image of a longitudinal imaging study of a patient. The lesion tracking framework is implemented using a machine learning based extraction network for extracting embeddings from the baseline medical image and the follow-up medical image associated with a plurality of scales. The extraction network may be trained using unlabeled (i.e., without lesion-related annotations) and unpaired (i.e., without the use of longitudinal medical images) training images. The locations of the lesions are determined in the follow-up medical image by comparing embeddings of the first set of embeddings and the second set of embeddings. Advantageously, by extracting embeddings associated with a plurality of scales, the extraction network processes increasingly finer information, starting from a coarse scale where the extraction network focuses on a larger region of the images, to one or more intermediate scales, and finally to a fine scale where the extraction network focuses on a more localized region to extract the most discriminative local features or embeddings. Further, the extraction network may be trained based on anatomical constraints to further enhance the extraction of the embeddings.

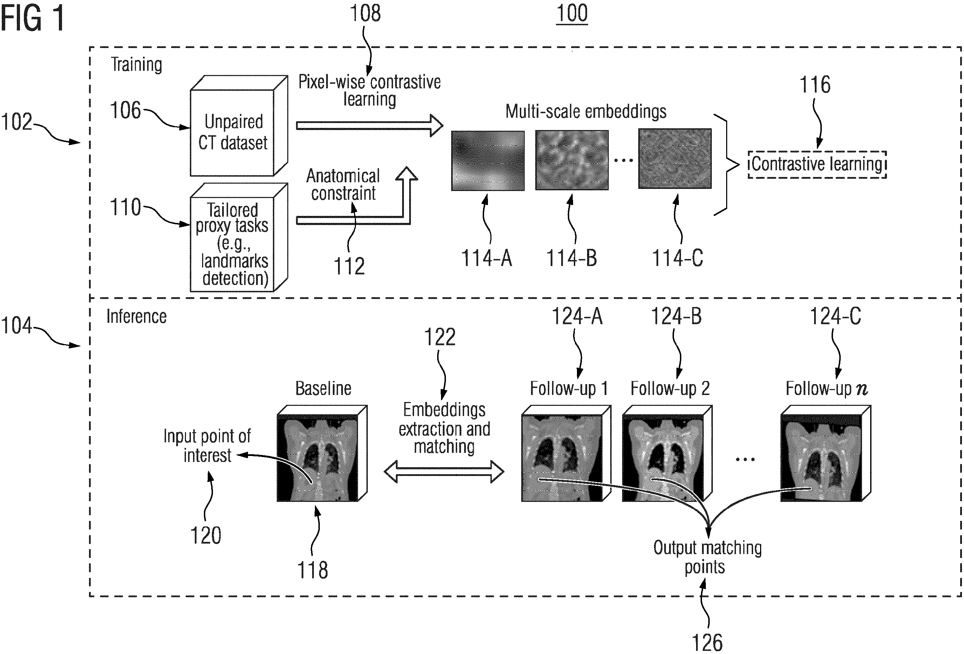

Figure 1 shows a framework 100 for training and applying a machine learning based extraction network for lesion tracking, in accordance with one or more embodiments. Framework 100 comprises a training stage 102 for training the extraction network and an inference stage 104 for applying the trained extraction network.

In training stage 102, the extraction network is trained based on dataset 106 of unpaired and unlabeled CT (computed tomography) images with pixel-wise contrastive learning 108 and, optionally, based on anatomical constraints 112 resulting from tailored proxy tasks 110 (e.g., landmark detection, segmentation, etc.) to generate a hierarchy of discriminative and fine-grained embeddings 114-A, 114-B, and 114-C at a plurality of scales. For example, embedding 114-A may be associated with a coarse scale, embedding 114-B may be associated with an intermediate scale, and embedding 114-C may be associated with a fine scale. The extraction network is trained with contrastive learning 116 to differentiate image pixels.

In inference stage 104, the trained extraction network separately receives as input baseline medical image 116 with input point of interest 120 corresponding to a location of a lesion in baseline medical image 116 and a follow-up medial image 124-A, 124-B, or 124-C. The trained extraction network separately performs embeddings extraction and matching 122 to generate output matching points 126, which have a location that correspond to the location of input point of interest 120, in follow-up medial image 124-A, 124-B, or 124-C.

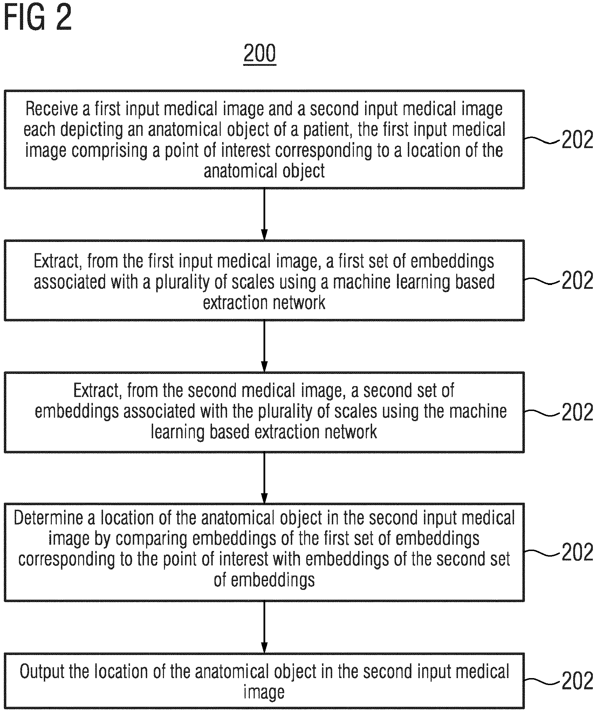

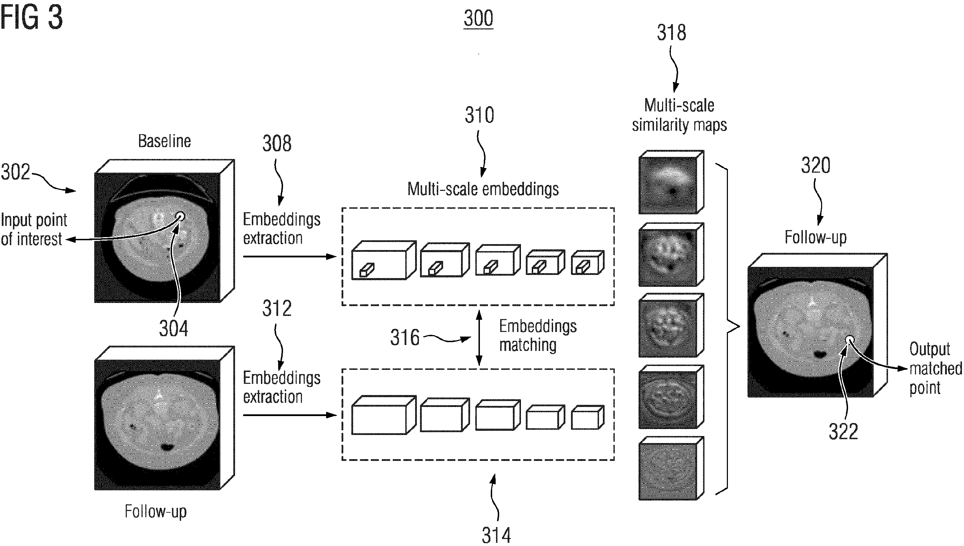

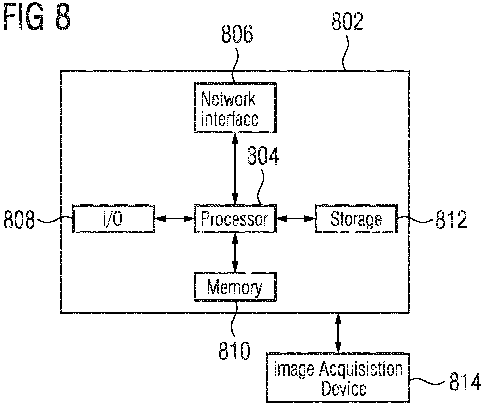

Figure 2 shows a method 200 for tracking an anatomical object in medical images using a machine learning based extraction network, in accordance with one or more embodiments. Figure 3 shows a workflow 300 for tracking an anatomical object in medical images using a machine learning based extraction network, in accordance with one or more embodiments. Figure 2 and Figure 3 will be described together. The steps of method 200 may be performed by one or more suitable computing devices, such as, e.g., computer 802 of Figure 8.

At step 202 of Figure 2, a first input medical image and a second input medical image each depicting an anatomical object of a patient is received. The first input medical image comprises a point of interest corresponding to a location of the anatomical object. In one embodiment, the anatomical object is a lesion. However, the anatomical object may be any other suitable anatomical object, such as, e.g., other abnormalities (e.g., tumors, nodules, etc.), organs, bones, anatomical landmarks, or any other anatomical object of interest.

The point of interest may be defined in any suitable manner. In one embodiment, the point of interest is defined by a user (e.g., a clinician) via user input received using, e.g., input/output device 808 of Figure 8. In another embodiment, the point of interest is automatically defined using a lesion detection system (e.g., a machine learning based lesion detection network).

In one embodiment, the first input medical image and the second input medical image respectively comprise a baseline medical image and a follow-up medical image each acquired from the patient at different points in time. For example, the baseline medical image and the follow-up medical image may be images of a longitudinal imaging study of the patient. In one example, as shown in workflow 300 of Figure 3, the first input medical image is baseline medical image 302 comprising point of interest 304 corresponding to a center location of a lesion and the second input medical image is follow-up medical image 306.

In one embodiment, the first input medical image and/or the second input medical image are CT images. However, the first input medical image and/or the second input medical image may comprise any other suitable modality, such as, e.g., MRI (magnetic resonance imaging), PET (positron emissions tomography), ultrasound, x-ray, or any other medical imaging modality or combinations of medical imaging modalities. The first input medical image and/or the second input medical image may be 2D (two dimensional) images and/or 3D (three dimensional) volumes, and may each comprise a single input medical image or a plurality of input medical images. The first input medical image and/or the second input medical image may be received directly from an image acquisition device, such as, e.g., a CT scanner, as the medical images are acquired, or can be received by loading previously acquired medical images from a storage or memory of a computer system or receiving medical images that have been transmitted from a remote computer system.

At step 204 of Figure 2, a first set of embeddings associated with a plurality of scales is extracted from the first input medical image using a machine learning based extraction network. At step 206 of Figure 2, a second set of embeddings associated with the plurality of scales is extracted from the second input medical image using the machine learning based extraction network. The plurality of scales comprises a coarse scale, one or more intermediate scales, and a fine scale.

In one example, as shown in workflow 300 of Figure 3, an extraction network performs embeddings extraction 308 by receiving as input baseline medical image 302 and generating as output a hierarchy of multi-scale embeddings 310. Separately, the extraction network performs embeddings extraction 312 by receiving as input follow-up medical image 306 and generating as output a hierarchy of multi-scale embeddings 314. In operation, the extraction network extracts regions of the input medical image (e.g., baseline medical image 302 or follow-up medical image 306) centered around each pixel (or voxel). The extracted regions have a scale according to one of the plurality of scales. The extraction network extracts a vector of embeddings from each of the extracted regions. The embeddings are latent features representing the most discriminative features of the regions as learned by the extraction network.

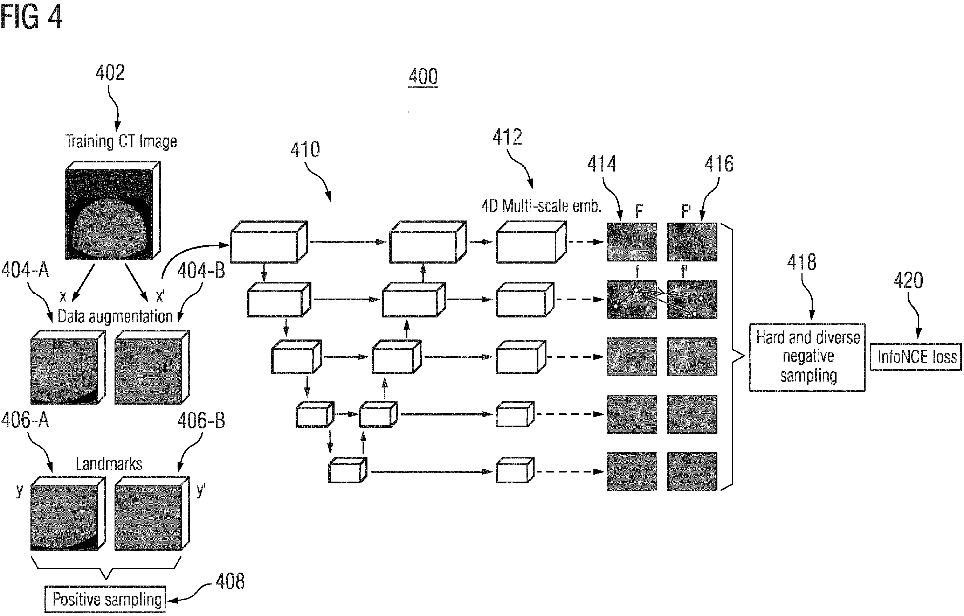

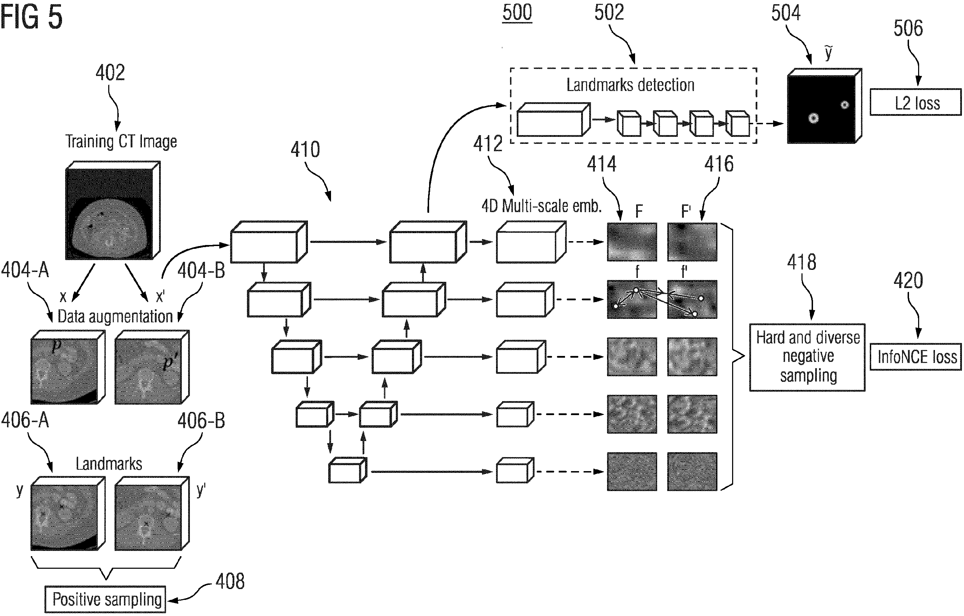

The extraction network may be any suitable machine learning based network for extracting the sets of embeddings from the first input medical image and the second input medical image. In one embodiment, the extraction network is an encoder network, e.g., of an autoencoder, a VAE (variational autoencoder), etc. The extraction network is trained during a prior training stage (e.g., training stage 102 of Figure 1). The extraction network may be trained using unlabeled and unpaired training images in an unsupervised or self-supervised approach, or may be trained using labelled training images in a semi-supervised or fully-supervised approach. In one embodiment, the extraction network is trained with positive sampling of detected anatomical landmarks, as further discussed below with respect to Figure 4. In one embodiment, the extraction network is jointly trained with another machine learning based network for performing a medical imaging analysis task (e.g., landmark detection), as further discussed below with respect to Figure 5. Once trained, the extraction network is applied at steps 204 and 206 of Figure 2 during an inference stage (or at, for example, inference stage 104 of Figure 1 or to perform embeddings extraction 308 and 312 in Figure 3) to respectively extract the first set of embeddings and the second set of embeddings from the first input medical image and the second input medical image.

At step 208 of Figure 2, a location of the anatomical object in the second input medical image is determined by comparing embeddings of the first set of embeddings corresponding to the point of interest with embeddings of the second set of embeddings. In one example, as shown in workflow 300 of Figure 3, the location of the lesion in follow-up medical image 206 is determined by performing embeddings matching 316. Embeddings matching 316 is performed by comparing multi-scale embeddings 310 that correspond to point of interest 304 with multi-scale embeddings 314. The comparison is performed according to a similarity measure. The similarity measure may be any suitable measure of vector similarity, such as, e.g., Euclidean distance, cosine similarity, dot product, etc.

The comparison of multi-scale embeddings 310 that correspond to point of interest 304 with multi-scale embeddings 314 is performed between embeddings with corresponding scales of the plurality of scales. For example, embeddings of multi-scale embeddings 310 and 314 corresponding to the coarse scale are compared with each other, embeddings of multi-scale embeddings 310 and 314 corresponding to the intermediate scale are compared with each other, and embeddings of multi-scale embeddings 310 and 314 corresponding to the fine scale are compared with each other. The comparison of multi-scale embeddings 310 and 314 results in a multi-scale similarity map 318 for each of the plurality of scales.

During training, similarity maps are being used to generate hard-negative sampling points (pairs of pixels from the two volumes that have similar embeddings but which shouldn't be matched). These hard-negative points, alongside true-positive (matched pixels) and negative points (unmatched pixels) are used to train the model via contrastive loss. Such points are extracted for each of the plurality of scales. During training the networks adapts the embeddings such that matched pixels have similar embeddings whereas unmatched pixels have different embeddings. Similarity maps 318 are generated by applying the, e.g., cosine operator between two embedding vectors. Having a point of interest 304 selected in the baseline medical image 302, the 4D embedding maps (that are iteratively adapted by the model during training) are used to extract the 3D vector embedding for that particular point of interest 304. The cosine similarity map is then computed between the 3D embedding vector of the baseline medical image 302 and each 3D vector of the 4D embedding maps of the follow-up medical image 306 to identify the pixels with greatest similarity (which are not true match) to use them as hard-negative samples during training.

Based on the comparison of multi-scale embeddings 310 and 314, matching embeddings of multi-scale embeddings 314 are identified that are most similar to the multi-scale embeddings 310 that correspond to point of interest 304. The similarity between the multi-scale embeddings 310 for the point of interest 304 in baseline medical image 302 and each embedding vector of multi-scale embeddings 314 extracted from follow-up medical image 306 is quantified by applying, e.g., the cosine operator. This procedure generates a cascade of cosine similarity maps which are merged trough summation and used to retrieve the prediction using the argmax operator. The location 322 of the lesion in the follow-up medical image 306 is determined as the pixel (or voxel) in follow-up medical image 306 that corresponds to the matching embeddings of multi-scale embeddings 314.

At step 210 of Figure 2, the location of the anatomical object in the second input medical image is output. For example, the location of the anatomical object in the second input medical image can be output by displaying the location of the anatomical object in the second input medical image on a display device of a computer system, storing the location of the anatomical object in the second input medical image on a memory or storage of a computer system, or by transmitting the location of the anatomical object in the second input medical image to a remote computer system. In one example, as shown in workflow 300 of Figure 3, the location of the anatomical object in the second input medical image may be output as an output image 320 comprising follow-up medical image 306 with location 322 of the lesion overlaid thereon is output.

The steps of method 200 of Figure 2 may be repeated for one or more iterations to respectively track the location of the anatomical object in one or more additional input medical images (e.g., one or more additional follow-up medical images in the longitudinal study). In one example, the steps of method 200 may be repeated using the second input medical image as the first input medical image and an additional input medical image as the second input medical image. In this manner, the location of the anatomical object is tracked from the first input medical image to the second input medical image during a first iteration of method 200, then from the second input medical image to the additional input medical image during a second iteration of method 200. In another example, the steps of method 200 may be repeated using an additional input medical image as the second input medical image. In this manner, the location of the anatomical object is tracked from the first input medical image to the second input medical image during a first iteration of method 200, then from the first input medical image to the additional input medical image during a second iteration of method 200.

Advantageously, by combining the information provided by multiple similarity maps 318 corresponding to different scales, the implications of misdetection caused by symmetry (e.g., pixels in the left and right kidneys) or by adjacent structures having similar appearances are reduced during the final fusion of the multi-scale similarity maps 318. This is especially useful when multiple lesions, for example, are closely positioned and the aim is to correctly match each lesion in the first input medical image with the corresponding lesion in the second input medical image in order to assess the changes in size and appearance throughout treatment of the lesions.

The extraction network (e.g., utilized at steps 204 and 206 of Figure 2 or at embeddings extraction 308 and 312 of Figure 3) may be trained with contrastive learning, such as, e.g., InfoNCE (noise contrastive estimation). Contrastive learning is used to differentiate image pixels by generating a hierarchy of discriminative and fine-grained embeddings without the use of pixel labels by relying solely on a sampling strategy that provides the necessary information to compute a loss function and adapt the parameters of the extraction network. Given a CT volume, paired 3D data are created on-the-fly by extracting sub-regions and applying random spatial and intensity-related transformations. During training, the extraction network learns to extract discriminative embeddings (same body parts will have similar encodings, being placed closer in the embedding space) that encode the appearance and anatomical context information of each image pixel.

Certain types of imaging (e.g., CT) provides contextual information about the patient that is naturally consistent. Therefore, the extraction network could benefit from biologically meaningful points (e.g., anatomical landmarks, segmentation masks, etc.) to further enhance the discriminativeness of the embeddings and ultimately the matching capabilities. In some embodiments, such anatomical landmarks may be incorporated into the training of the extraction networks at two levels: 1) directly into the sampling procedure (as further discussed with respect to Figure 4 below) and/or 2) as an additional learned task (as further discussed with respect to Figure 5 below).

Figure 4 shows a workflow 400 for training a machine learning based extraction network, in accordance with one or more embodiments. Workflow 400 is performed during a prior training stage (e.g., training stage 102 of Figure 1). Once trained, the trained extraction network may be applied during an inference stage (e.g., inference stage 104 of Figure 1, steps 204 and 206 of Figure 2, embeddings extraction 308 and 312 of Figure 3). Depending on the availability of annotations on the training dataset (e.g., training CT image 402), workflow 400 provides for the training of the extraction network in a self-supervised approach (i.e., no annotations required), a semi-supervised approach (i.e., some cases have annotations), or a fully supervised approach (i.e., all cases have annotations).

In workflow 400, the extraction network is trained using training CT image 402. In one embodiment, in a scenario where training CT image 402 is annotated for, e.g., lesions, landmarks, etc., annotated training CT image 402 may be leveraged to enable positive sampling 408. Synthetic pair of images 404-A and 404-B (referred to herein as synthetic pair 404) and synthetic pair of images 406-A and 406-B (referred to herein as synthetic pair 406) are generated from annotated training CT image 402 via data augmentation by applying one or more affine transformations (e.g., rotation, translation, scaling, or any other spatial or intensity-related transformation) to training CT image 402. Image $x$ 404-A and image $y$ 406-A are training CT image 402 while augmented image $x'$ 404-B and augmented image $y'$ 406-B are generated by applying the one or more transformations of training CT image 402. Image $x$ 404-A is labeled or annotated to identify a point of interest $p$ corresponding to a location of a lesion. Image $y$ 406-A is labeled to identify one or more anatomical landmarks. Such lesions and anatomical landmarks may be manually identified by a user or automatically identified by, e.g., a machine learning based detection network. Since the transformations applied to training CT image 402 for generating augmented image $x'$ 406-B and augmented image $y'$ 406-B is known, point of interest $p'$ corresponding to a location of the lesion in augmented image $x'$ 404-B and the locations of the one or more anatomical landmarks in augmented image $y'$ 406-B are also known. In this manner, the extraction network is trained with positive sampling 408 with synthetically generated pairs of labeled images.

In one embodiment, annotated training CT image 402 may be leveraged to enable a hard and diverse negative sampling 418. In this embodiment, point of interest $p'$ in augmented image $x'$ 404-B may be selected as any other (e.g., arbitrary) point that does not correspond to point of interest $p$ in image $x$ 404-A and the locations of the one or more anatomical landmarks in augmented image $y'$ 406-B may be selected as any other (e.g., arbitrary) point that does not correspond to the anatomical landmarks in image y 406-A.

In one embodiment, where a segmentation mask of, e.g., the lesions or the anatomical landmarks, is available, positive sampling 408 and negative sampling 418 of pixels can be performed from the segmented region based on a distance map. Distance maps obtained from ground truth masks can be used to guide pairs sampling towards the inner regions. This approach seeks to generate a distance map in which pixels close to the centroid segmentation or centerline (i.e., skeletonization) are weighted more heavily than those further away.

In one embodiment, the extraction network is trained in a self-supervised approach without annotated training data. In this embodiment, one or more points of interest $p$ are randomly selected from an overlapping area between image 404-A and augmented image $x'$ 404-B. For each point of interest $p$ from image 404-A, the corresponding point of interest $p'$ in augmented image $x'$ 404-B is identified since the affine transformations that were performed to generate augmented image $x'$ 404-B is known. These matched points of interest $p$ and $p'$ define the points for positive sampling 408 for which the extraction network needs to minimize the distance between embeddings.

Synthetic pairs 404 and 406 are input into layers 410 of the extraction network for extracting 4D multi-scale embeddings 412 at a plurality of scales and respective similarity maps $F$ 414 and $F'$ 416 are generated for synthetic pairs 404 and 406. The extraction network is trained with a contrastive loss, such as, e.g., InfoNCE (noise contrastive estimation) loss 420. Given the nature of contrastive learning, the sampling strategy (extracting negative and positive pixel pairs from augmented 3D paired patches) is important to achieving discriminative embeddings. Hence, having access to specific landmarks enables the sampling of positive pixel samples across different volumes (i.e., same landmark different volume). The reasoning behind this strategy is that simple data augmentation methods cannot faithfully model inter-subject variability or possible organ deformations.

Figure 5 shows a workflow 500 for training a machine learning based extraction network with an auxiliary task, in accordance with one or more embodiments. Workflow 500 is performed during a prior training stage (e.g., training stage 102 of Figure 1). Once trained, the trained extraction network may be applied during an inference stage (e.g., inference stage 104 of Figure 1, steps 204 and 206 of Figure 2, embeddings extraction 308 and 312 of Figure 3).

In workflow 500, the extraction network is trained with multi-task learning to additionally perform one or more auxiliary task, such as, e.g., landmark regression, organ/pathology segmentations, pixel-wise matching, image reconstruction. As shown in Figure 5, the extraction network comprises layers 502 for generating heat map $\tilde{y}$ 504 to perform landmark detection. Layers 502 are trained with L2 loss 506. The auxiliary tasks allow the network to better capture the underlying patterns of human anatomy and focus the training on a specific direction by providing prior knowledge that can benefit the main task of embeddings extraction. Advantageously, by incorporating anatomical landmark constraints into the training of the extraction network by means of supervision, the extraction network learns to distinguish between lesions with similar appearances.

While embodiments described herein are described with respect to tracking lesions (or other anatomical objects) from a first input medical image to a second input medical image, the present invention is not so limited. The embeddings (e.g., extracted at steps 204 and 206 of Figure 2) may be utilized to perform other tasks, such s, e.g., landmark detection, registration, and network pretraining for other tasks (e.g., segmentation, object detection).

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained machine learning based networks (or models), as well as with respect to methods and systems for training machine learning based networks. Features, advantages or alternative embodiments herein can be assigned to the other claimed objects and vice versa. In other words, claims for methods and systems for training a machine learning based network can be improved with features described or claimed in context of the methods and systems for utilizing a trained machine learning based network, and vice versa.

In particular, the trained machine learning based networks applied in embodiments described herein can be adapted by the methods and systems for training the machine learning based networks. Furthermore, the input data of the trained machine learning based network can comprise advantageous features and embodiments of the training input data, and vice versa. Furthermore, the output data of the trained machine learning based network can comprise advantageous features and embodiments of the output training data, and vice versa.

In general, a trained machine learning based network mimics cognitive functions that humans associate with other human minds. In particular, by training based on training data, the trained machine learning based network is able to adapt to new circumstances and to detect and extrapolate patterns.

In general, parameters of a machine learning based network can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based network can be adapted iteratively by several steps of training.

In particular, a trained machine learning based network can comprise a neural network, a support vector machine, a decision tree, and/or a Bayesian network, and/or the trained machine learning based network can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

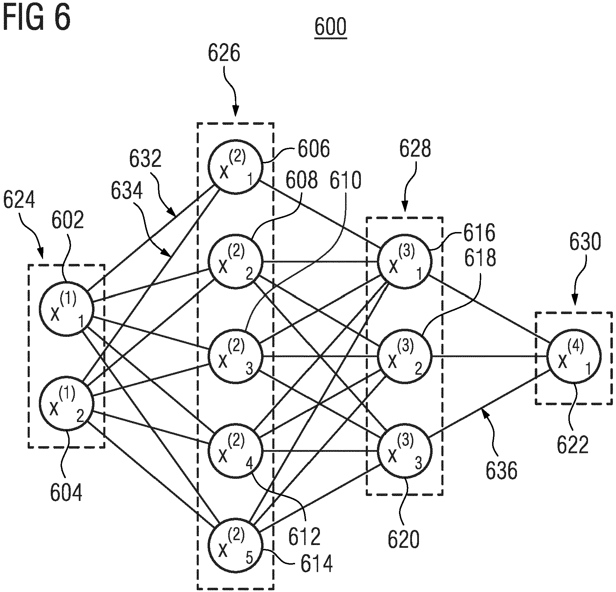

Figure 6 shows an embodiment of an artificial neural network 600, in accordance with one or more embodiments. Alternative terms for "artificial neural network" are "neural network", "artificial neural net" or "neural net". Machine learning networks described herein, such as, e.g., the extraction network trained and applied in Figure 1, the extraction network utilized at steps 204 and 206 of Figure 2, the extraction network utilized to perform embeddings extraction 308 and 312 of Figure 3, the extraction network trained according to Figure 4, and the extraction network trained according to Figure 5, may be implemented using artificial neural network 600.

The artificial neural network 600 comprises nodes 602-622 and edges 632, 634, ..., 636, wherein each edge 632, 634, ..., 636 is a directed connection from a first node 602-622 to a second node 602-622. In general, the first node 602-622 and the second node 602-622 are different nodes 602-622, it is also possible that the first node 602-622 and the second node 602-622 are identical. For example, in Figure 6, the edge 632 is a directed connection from the node 602 to the node 606, and the edge 634 is a directed connection from the node 604 to the node 606. An edge 632, 634, ..., 636 from a first node 602-622 to a second node 602-622 is also denoted as "ingoing edge" for the second node 602-622 and as "outgoing edge" for the first node 602-622.

In this embodiment, the nodes 602-622 of the artificial neural network 600 can be arranged in layers 624-630, wherein the layers can comprise an intrinsic order introduced by the edges 632, 634, ..., 636 between the nodes 602-622. In particular, edges 632, 634, ..., 636 can exist only between neighboring layers of nodes. In the embodiment shown in Figure 6, there is an input layer 624 comprising only nodes 602 and 604 without an incoming edge, an output layer 630 comprising only node 622 without outgoing edges, and hidden layers 626, 628 in-between the input layer 624 and the output layer 630. In general, the number of hidden layers 626, 628 can be chosen arbitrarily. The number of nodes 602 and 604 within the input layer 624 usually relates to the number of input values of the neural network 600, and the number of nodes 622 within the output layer 630 usually relates to the number of output values of the neural network 600.

In particular, a (real) number can be assigned as a value to every node 602-622 of the neural network 600. Here, $x^{(n)}_i$ denotes the value of the i-th node 602-622 of the n-th layer 624-630. The values of the nodes 602-622 of the input layer 624 are equivalent to the input values of the neural network 600, the value of the node 622 of the output layer 630 is equivalent to the output value of the neural network 600. Furthermore, each edge 632, 634, ..., 636 can comprise a weight being a real number, in particular, the weight is a real number within the interval [-1, 1] or within the interval [0, 1]. Here, $w^{(m,n)}_{i,j}$ denotes the weight of the edge between the i-th node 602-622 of the m-th layer 624-630 and the j-th node 602-622 of the n-th layer 624-630. Furthermore, the abbreviation $w^{(n)}_{i,j}$ is defined for the weight $w^{(n,n+1)}_{i,j}$.

In particular, to calculate the output values of the neural network 600, the input values are propagated through the neural network. In particular, the values of the nodes 602-622 of the (n+1)-th layer 624-630 can be calculated based on the values of the nodes 602-622 of the n-th layer 624-630 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 624 are given by the input of the neural network 600, wherein values of the first hidden layer 626 can be calculated based on the values of the input layer 624 of the neural network, wherein values of the second hidden layer 628 can be calculated based in the values of the first hidden layer 626, etc.

In order to set the values $w^{(m,n)}_{i,j}$ for the edges, the neural network 600 has to be trained using training data. In particular, training data comprises training input data and training output data (denoted as $t_i$). For a training step, the neural network 600 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 600 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein y is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta^{(n)}_j = \left(x^{(n+1)}_k - t^{(n+1)}_j\right) \cdot f'\left(\sum_i x^{(n)}_i \cdot w^{(n)}_{i,j}\right)$$

if the (n+1)-th layer is the output layer 630, wherein f is the first derivative of the activation function, and $y^{(n+1)}_j$ is the comparison training value for the j-th node of the output layer 630.

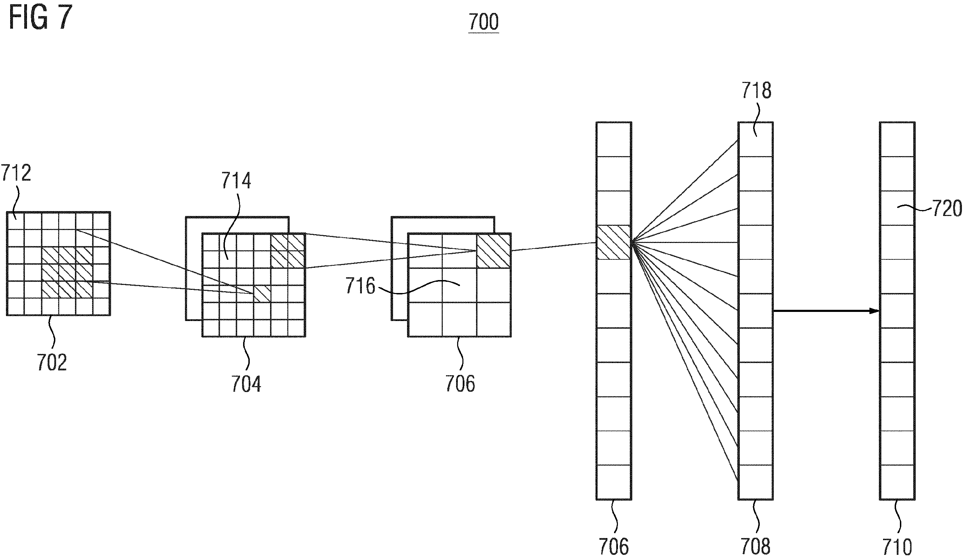

Figure 7 shows a convolutional neural network 700, in accordance with one or more embodiments. Machine learning networks described herein, such as, e.g., the extraction network trained and applied in Figure 1, the extraction network utilized at steps 204 and 206 of Figure 2, the extraction network utilized to perform embeddings extraction 308 and 312 of Figure 3, the extraction network trained according to Figure 4, and the extraction network trained according to Figure 5, may be implemented using convolutional neural network 700.

In the embodiment shown in Figure 7, the convolutional neural network comprises 700 an input layer 702, a convolutional layer 704, a pooling layer 706, a fully connected layer 708, and an output layer 710. Alternatively, the convolutional neural network 700 can comprise several convolutional layers 704, several pooling layers 706, and several fully connected layers 708, as well as other types of layers. The order of the layers can be chosen arbitrarily, usually fully connected layers 708 are used as the last layers before the output layer 710.

In particular, within a convolutional neural network 700, the nodes 712-720 of one layer 702-710 can be considered to be arranged as a d-dimensional matrix or as a d-dimensional image. In particular, in the two-dimensional case the value of the node 712-720 indexed with i and j in the n-th layer 702-710 can be denoted as $x^{(n)}{}_{[i,j]}$. However, the arrangement of the nodes 712-720 of one layer 702-710 does not have an effect on the calculations executed within the convolutional neural network 700 as such, since these are given solely by the structure and the weights of the edges.

In particular, a convolutional layer 704 is characterized by the structure and the weights of the incoming edges forming a convolution operation based on a certain number of kernels. In particular, the structure and the weights of the incoming edges are chosen such that the values $x^{(n)}{}_k$ of the nodes 714 of the convolutional layer 704 are calculated as a convolution $x^{(n)}{}_k = K_k * x^{(n-1)}$ based on the values $x^{(n-1)}$ of the nodes 712 of the preceding layer 702, where the convolution * is defined in the two-dimensional case as

$$x_k^{(n)}[i,j] = \left(K_k * x^{(n-1)}\right)[i,j] = \sum_{i'} \sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 712-718 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 712-720 in the respective layer 702-710. In particular, for a convolutional layer 704, the number of nodes 714 in the convolutional layer is equivalent to the number of nodes 712 in the preceding layer 702 multiplied with the number of kernels.

If the nodes 712 of the preceding layer 702 are arranged as a d-dimensional matrix, using a plurality of kernels can be interpreted as adding a further dimension (denoted as "depth" dimension), so that the nodes 714 of the convolutional layer 704 are arranged as a (d+1)-dimensional matrix. If the nodes 712 of the preceding layer 702 are already arranged as a (d+1)-dimensional matrix comprising a depth dimension, using a plurality of kernels can be interpreted as expanding along the depth dimension, so that the nodes 714 of the convolutional layer 704 are arranged also as a (d+1)-dimensional matrix, wherein the size of the (d+1)-dimensional matrix with respect to the depth dimension is by a factor of the number of kernels larger than in the preceding layer 702.

The advantage of using convolutional layers 704 is that spatially local correlation of the input data can exploited by enforcing a local connectivity pattern between nodes of adjacent layers, in particular by each node being connected to only a small region of the nodes of the preceding layer.

In embodiment shown in Figure 7, the input layer 702 comprises 36 nodes 712, arranged as a two-dimensional 6x6 matrix. The convolutional layer 704 comprises 72 nodes 714, arranged as two two-dimensional 6x6 matrices, each of the two matrices being the result of a convolution of the values of the input layer with a kernel. Equivalently, the nodes 714 of the convolutional layer 704 can be interpreted as arranges as a three-dimensional 6x6x2 matrix, wherein the last dimension is the depth dimension.

A pooling layer 706 can be characterized by the structure and the weights of the incoming edges and the activation function of its nodes 716 forming a pooling operation based on a non-linear pooling function f. For example, in the two dimensional case the values $x^{(n)}$ of the nodes 716 of the pooling layer 706 can be calculated based on the values $x^{(n-1)}$ of the nodes 714 of the preceding layer 704 as

$$x^{(n)}[i,j] = f\left(x^{(n-1)}[id_1, jd_2], \ldots, x^{(n-1)}[id_1+d_1-1, jd_2+d_2-1]\right)$$

In other words, by using a pooling layer 706, the number of nodes 714, 716 can be reduced, by replacing a number $d1 \cdot d2$ of neighboring nodes 714 in the preceding layer 704 with a single node 716 being calculated as a function of the values of said number of neighboring nodes in the pooling layer. In particular, the pooling function f can be the max-function, the average or the L2-Norm. In particular, for a pooling layer 706 the weights of the incoming edges are fixed and are not modified by training.

The advantage of using a pooling layer 706 is that the number of nodes 714, 716 and the number of parameters is reduced. This leads to the amount of computation in the network being reduced and to a control of overfitting.

In the embodiment shown in Figure 7, the pooling layer 706 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 708 can be characterized by the fact that a majority, in particular, all edges between nodes 716 of the previous layer 706 and the nodes 718 of the fully-connected layer 708 are present, and wherein the weight of each of the edges can be adjusted individually.

In this embodiment, the nodes 716 of the preceding layer 706 of the fully-connected layer 708 are displayed both as two-dimensional matrices, and additionally as non-related nodes (indicated as a line of nodes, wherein the number of nodes was reduced for a better presentability). In this embodiment, the number of nodes 718 in the fully connected layer 708 is equal to the number of nodes 716 in the preceding layer 706. Alternatively, the number of nodes 716, 718 can differ.

Furthermore, in this embodiment, the values of the nodes 720 of the output layer 710 are determined by applying the Softmax function onto the values of the nodes 718 of the preceding layer 708. By applying the Softmax function, the sum the values of all nodes 720 of the output layer 710 is 1, and all values of all nodes 720 of the output layer are real numbers between 0 and 1.

A convolutional neural network 700 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 700 can be trained based on the backpropagation algorithm. For preventing overfitting, methods of regularization can be used, e.g. dropout of nodes 712-720, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-5. Certain steps or functions of the methods and workflows described herein, including one or more of the steps or functions of Figures 1-5, may be performed by a server or by another processor in a network-based cloud-computing system. Certain steps or functions of the methods and workflows described herein, including one or more of the steps of Figures 1-5, may be performed by a client computer in a network-based cloud computing system. The steps or functions of the methods and workflows described herein, including one or more of the steps of Figures 1-5, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-5, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 802 that may be used to implement systems, apparatus, and methods described herein is depicted in Figure 8. Computer 802 includes a processor 804 operatively coupled to a data storage device 812 and a memory 810. Processor 804 controls the overall operation of computer 802 by executing computer program instructions that define such operations. The computer program instructions may be stored in data storage device 812, or other computer readable medium, and loaded into memory 810 when execution of the computer program instructions is desired. Thus, the method and workflow steps or functions of Figures 1-5 can be defined by the computer program instructions stored in memory 810 and/or data storage device 812 and controlled by processor 804 executing the computer program instructions. For example, the computer program instructions can be implemented as computer executable code programmed by one skilled in the art to perform the method and workflow steps or functions of Figures 1-5.

Accordingly, by executing the computer program instructions, the processor 804 executes the method and workflow steps or functions of Figures 1-5. Computer 802 may also include one or more network interfaces 806 for communicating with other devices via a network. Computer 802 may also include one or more input/output devices 808 that enable user interaction with computer 802 (e.g., display, keyboard, mouse, speakers, buttons, etc.).

Processor 804 may include both general and special purpose microprocessors, and may be the sole processor or one of multiple processors of computer 802. Processor 804 may include one or more central processing units (CPUs), for example. Processor 804, data storage device 812, and/or memory 810 may include, be supplemented by, or incorporated in, one or more application-specific integrated circuits (ASICs) and/or one or more field programmable gate arrays (FPGAs).

Data storage device 812 and memory 810 each include a tangible non-transitory computer readable storage medium. Data storage device 812, and memory 810, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 808 may include peripherals, such as a printer, scanner, display screen, etc. For example, input/output devices 808 may include a display device such as a cathode ray tube (CRT) or liquid crystal display (LCD) monitor for displaying information to the user, a keyboard, and a pointing device such as a mouse or a trackball by which the user can provide input to computer 802.

An image acquisition device 814 can be connected to the computer 802 to input image data (e.g., medical images) to the computer 802. It is possible to implement the image acquisition device 814 and the computer 802 as one device. It is also possible that the image acquisition device 814 and the computer 802 communicate wirelessly through a network. In a possible embodiment, the computer 802 can be located remotely with respect to the image acquisition device 814.

Any or all of the systems and apparatus discussed herein may be implemented using one or more computers such as computer 802.

One skilled in the art will recognize that an implementation of an actual computer or computer system may have other structures and may contain other components as well, and that Figure 8 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention.

**Claims**

1. A computer implemented method comprising:

   receiving (202) a first input medical image (118, 302) and a second input medical image (124-A, 306) each depicting an anatomical object of a patient, the first input medical image comprising a point of interest (120, 304) corresponding to a location of the anatomical object;

extracting (204), from the first input medical image, a first set of embeddings (310) associated with a plurality of scales using a machine learning based extraction network, the plurality of scales comprising a coarse scale, one or more intermediate scales, and a fine scale;

extracting (206), from the second input medical image, a second set of embeddings (314) associated with the plurality of scales using the machine learning based extraction network;

determining (208) a location (126, 322) of the anatomical object in the second input medical image by comparing (316) embeddings of the first set of embeddings corresponding to the point of interest with embeddings of the second set of embeddings; and

outputting (210) the location of the anatomical object in the second input medical image.

2. The computer implemented method of claim 1, wherein the machine learning based extraction network is trained based on anatomical landmarks identified in a pair of training images (404-A and 404-B, 406-A and 406-B).

3. The computer implemented method of claim 1 or 2, wherein the machine learning based extraction network is trained with multi-task learning to perform one or more auxiliary tasks.

4. The computer implemented method of claim 3, wherein the one or more auxiliary tasks comprise at least one of landmark detection, segmentation, pixel-wise matching, or image reconstruction.

5. The computer implemented method of any one of claims 1 - 4, wherein the machine learning based extraction network is trained with unlabeled and unpaired training images.

6. The computer implemented method of any one of claims 1 - 5, wherein determining a location of the anatomical object in the second input medical image by comparing embeddings of the first set of embeddings corresponding to the point of interest with embeddings of the second set of embeddings comprises:

identifying matching embeddings of the second set of embeddings that are most similar to embeddings of the first set of embeddings corresponding to the point of interest; and

determining the location of the anatomical object in the second input medical image as a pixel or voxel that corresponds to the matching embeddings.

7. The computer implemented method of any one of claims 1 - 6, wherein determining a location of the anatomical object in the second input medical image by comparing embeddings of the first set of embeddings corresponding to the point of interest with embeddings of the second set of embeddings comprises:

comparing embeddings of the first set of embeddings and the second set of embeddings with corresponding scales of the plurality of scales.

8. The computer implemented method of any one of claims 1 - 7, further comprising:

repeating the receiving, the extracting the first set of embeddings, the extracting the second set of embeddings, the determining, and the outputting using an additional input medical image (124-B, 124-C) as the second input medical image.

9. The computer implemented method of any one of claims 1 - 8, wherein the first input medical image and the second input medical image respectively comprise a baseline medical image and a follow-up medical image of a longitudinal imaging study of the patient.

10. An apparatus comprising:

means for receiving (202) a first input medical image (118, 302) and a second input medical image (124-A, 306) each depicting an anatomical object of a patient, the first input medical image comprising a point of interest (120, 304) corresponding to a location of the anatomical object;

means for extracting (204), from the first input medical image, a first set of embeddings (310) associated with a plurality of scales using a machine learning based extraction network, the plurality of scales comprising a coarse scale, one or more intermediate scales, and a fine scale;

means for extracting (206), from the second input medical image, a second set of embeddings (314) associated with the plurality of scales using the machine learning based extraction network;

means for determining (208) a location (126, 322) of the anatomical object in the second input medical image by comparing (316) embeddings of the first set of embeddings corresponding to the point of interest with embeddings of the second set of embeddings; and

means for outputting (210) the location of the anatomical object in the second input medical image.

11. The apparatus of claim 10, wherein the means are implemented to carry out the mehtod of any one of claims 1 - 9.

12. A non-transitory computer readable medium storing computer program instructions, the computer program instructions when executed by a processor cause the processor to perform operations comprising:

receiving (202) a first input medical image (118, 302) and a second input medical image (124-A, 306) each depicting an anatomical object of a patient, the first input medical image comprising a point of interest (120, 304) corresponding to a location of the anatomical object;

extracting (204), from the first input medical image, a first set of embeddings (310) associated with a plurality of scales using a machine learning based extraction network, the plurality of scales comprising a coarse scale, one or more intermediate scales, and a fine scale;

extracting (206), from the second input medical image, a second set of embeddings (314) associated with the plurality of scales using the machine learning based extraction network;

determining (208) a location (126, 322) of the anatomical object in the second input medical image by comparing (316) embeddings of the first set of embeddings corresponding to the point of interest with embeddings of the second set of embeddings; and

outputting (210) the location of the anatomical object in the second input medical image.

13. The non-transitory computer readable medium of claim 15, wherein the computer program instructions when executed by the processor cause the processor to perform operations comprising the method of any one of claims 1 - 9.

**Amended claims in accordance with Rule 137(2) EPC.**

1. A computer implemented method comprising:

receiving (202) a first input medical image (118, 302) and a second input medical image (124-A, 306) each depicting an anatomical object of a patient, the first input medical image comprising a point of interest (120, 304) corresponding to a location of the anatomical object;

extracting (204), from the first input medical image, a first set of embeddings (310) associated with a plurality of scales using a machine learning based extraction network, the plurality of scales comprising a coarse scale, one or more intermediate scales, and a fine scale;

extracting (206), from the second input medical image, a second set of embeddings (314) associated with the plurality of scales using the machine learning based extraction network;

determining (208) a location (126, 322) of the anatomical object in the second input medical image by comparing (316) the embeddings of the first set of embeddings corresponding to the point of interest with the embeddings of the second set of embeddings; and

outputting (210) the location of the anatomical object in the second input medical image.

2. The computer implemented method of claim 1, wherein the machine learning based extraction network is trained based on anatomical landmarks identified in a pair of training images (404-A and 404-B, 406-A and 406-B).

3. The computer implemented method of claim 1 or 2, wherein the machine learning based extraction network is trained with multi-task learning to perform one or more auxiliary tasks.

4. The computer implemented method of claim 3, wherein the one or more auxiliary tasks comprise at least one of landmark detection, segmentation, pixelwise matching, or image reconstruction.

5. The computer implemented method of any one of claims 1-4, wherein the machine learning based extraction network is trained with unlabeled and unpaired training images.

6. The computer implemented method of any one of claims 1-5, wherein determining a location of the anatomical object in the second input medical image by comparing embeddings of the first set of embeddings corresponding to the point of interest with embeddings of the second set of embeddings comprises:

> identifying matching embeddings of the second set of embeddings that are most similar to embeddings of the first set of embeddings corresponding to the point of interest; and
> determining the location of the anatomical object in the second input medical image as a pixel or voxel that corresponds to the matching embeddings.

**7.** The computer implemented method of any one of claims 1-6, wherein determining a location of the anatomical object in the second input medical image by comparing embeddings of the first set of embeddings corresponding to the point of interest with embeddings of the second set of embeddings comprises:
comparing embeddings of the first set of embeddings and the second set of embeddings with corresponding scales of the plurality of scales.

**8.** The computer implemented method of any one of claims 1-7, further comprising:
repeating the receiving, the extracting the first set of embeddings, the extracting the second set of embeddings, the determining, and the outputting using an additional input medical image (124-B, 124-C) as the second input medical image.

**9.** The computer implemented method of any one of claims 1-8, wherein the first input medical image and the second input medical image respectively comprise a baseline medical image and a follow-up medical image of a longitudinal imaging study of the patient.

**10.** An apparatus comprising:

> means for receiving (202) a first input medical image (118, 302) and a second input medical image (124-A, 306) each depicting an anatomical object of a patient, the first input medical image comprising a point of interest (120, 304) corresponding to a location of the anatomical object;
> means for extracting (204), from the first input medical image, a first set of embeddings (310) associated with a plurality of scales using a machine learning based extraction network, the plurality of scales comprising a coarse scale, one or more intermediate scales, and a fine scale;
> means for extracting (206), from the second input medical image, a second set of embeddings (314) associated with the plurality of scales using the machine learning based extraction network;
> means for determining (208) a location (126, 322) of the anatomical object in the second input medical image by comparing (316) the embeddings of the first set of embeddings corresponding to the point of interest with the embeddings of the second set of embeddings; and
> means for outputting (210) the location of the anatomical object in the second input medical image.

**11.** The apparatus of claim 10, wherein the means are implemented to carry out the method of any one of claims 1 - 9.

**12.** A non-transitory computer readable medium storing computer program instructions, the computer program instructions when executed by a processor cause the processor to perform operations comprising:

> receiving (202) a first input medical image (118, 302) and a second input medical image (124-A, 306) each depicting an anatomical object of a patient, the first input medical image comprising a point of interest (120, 304) corresponding to a location of the anatomical object;
> extracting (204), from the first input medical image, a first set of embeddings (310) associated with a plurality of scales using a machine learning based extraction network, the plurality of scales comprising a coarse scale, one or more intermediate scales, and a fine scale;
> extracting (206), from the second input medical image, a second set of embeddings (314) associated with the plurality of scales using the machine learning based extraction network;
> determining (208) a location (126, 322) of the anatomical object in the second input medical image by comparing (316) the embeddings of the first set of embeddings corresponding to the point of interest with the embeddings of the second set of embeddings; and
> outputting (210) the location of the anatomical object in the second input medical image.

**13.** The non-transitory computer readable medium of claim 12, wherein the computer program instructions when executed by the processor cause the processor to perform operations comprising the method of any one of claims 1-9.

FIG 1

# FIG 2

200

```
┌─────────────────────────────────────────────────────────────┐
│ Receive a first input medical image and a second input medical image │
│ each depicting an anatomical object of a patient, the first input medical │──202
│ image comprising a point of interest corresponding to a location of the │
│                    anatomical object                         │
└─────────────────────────────────────────────────────────────┘
                              │
                              ▼
┌─────────────────────────────────────────────────────────────┐
│ Extract, from the first input medical image, a first set of embeddings │
│ associated with a plurality of scales using a machine learning based │──202
│                    extraction network                        │
└─────────────────────────────────────────────────────────────┘
                              │
                              ▼
┌─────────────────────────────────────────────────────────────┐
│ Extract, from the second medical image, a second set of      │
│ embeddings associated with the plurality of scales using the machine │──202
│              learning based extraction network               │
└─────────────────────────────────────────────────────────────┘
                              │
                              ▼
┌─────────────────────────────────────────────────────────────┐
│ Determine a location of the anatomical object in the second input medical │
│ image by comparing embeddings of the first set of embeddings │──202
│ corresponding to the point of interest with embeddings of the second set │
│                    of embeddings                             │
└─────────────────────────────────────────────────────────────┘
                              │
                              ▼
┌─────────────────────────────────────────────────────────────┐
│ Output the location of the anatomical object in the second input medical │──202
│                         image                                │
└─────────────────────────────────────────────────────────────┘
```

# FIG 3

EP 4 375 927 A1

# FIG 4

Training CT Image 402

400

Data augmentation
x 404-A     x' 404-B
p 406-A     p' 406-B

Landmarks
y           y'

Positive sampling 408

410

4D Multi-scale emb. 412

F 414     F' 416

f         f'

Hard and diverse negative sampling 418

InfoNCE loss 420

EP 4 375 927 A1

FIG 5

FIG 6

FIG 7

700

712

714

718

702

704

716

706

706

708

720

710

FIG 8

806      802

Network interface

804

808   I/O  ↔  Processor  ↔  Storage   812

Memory

810

Image Acquisistion Device   814

Europäisches Patentamt
European Patent Office
Office européen des brevets

# EUROPEAN SEARCH REPORT

Application Number

EP 22 20 9876

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MENGWEI REN ET AL: "Local Spatiotemporal Representation Learning for Longitudinally-consistent Neuroimage Analysis", ARXIV.ORG, CORNELL UNIVERSITY LIBRARY, 201 OLIN LIBRARY CORNELL UNIVERSITY ITHACA, NY 14853, 27 November 2022 (2022-11-27), XP091378653, * abstract * * sections 1 and 3 * * figures 1, 2, 3, 5 * | 1-13 | INV. G06T7/33 G06T7/35 ADD. A61B6/03 |
| X | JINZHENG CAI ET AL: "Deep Lesion Tracker: Monitoring Lesions in 4D Longitudinal Imaging Studies", ARXIV.ORG, CORNELL UNIVERSITY LIBRARY, 201 OLIN LIBRARY CORNELL UNIVERSITY ITHACA, NY 14853, 12 April 2021 (2021-04-12), XP081927385, * abstract * * sections 1, 3, 4 * * figures 1, 2 * | 1-13 | |
| X | US 2022/180126 A1 (YAN KE [US] ET AL) 9 June 2022 (2022-06-09) * paragraph [0024] - paragraph [0052] * * figures 1, 3, 4 * | 1-13 | TECHNICAL FIELDS SEARCHED (IPC) G06T A61B |

The present search report has been drawn up for all claims

| Place of search | Date of completion of the search | Examiner |
|---|---|---|
| Munich | 11 May 2023 | Gitzenis, Savvas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 9876

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2023

| Patent document cited in search report | Publication date | Patent family member(s) | | Publication date |
|---|---|---|---|---|
| US 2022180126 A1 | 09-06-2022 | US | 2022180126 A1 | 09-06-2022 |
| | | WO | 2022116869 A1 | 09-06-2022 |

For more details about this annex : see Official Journal of the European Patent Office, No. 12/82